# FASCICULE DE BREVET EUROPEEN

(11) **EP 0 507 076 B1**
(45) Date de publication et mention de la délivrance du brevet: **21.06.1995**
(21) Numéro de dépôt: 92103221.5
(22) Date de dépôt: 26.02.1992
(51) Int. Cl.: C07C 43/04, C07C 41/42, C07C 41/06, C07C 31/08, C07C 29/82

(54) **Procédé de séparation d'éthyltertiobutyléther à partir de mélanges avec l'éthanol**
Verfahren zur Trennung von Ehtyl-tert-butylether aus Ethanol enthaltenden Mischungen
Method for separating ethyl tert-butyl ether from mixtures with ethanol

(30) Priorité: 07.03.1991 FR 9102893
(43) Date de publication de la demande: 07.10.1992
(73) Titulaire: INSTITUT FRANCAIS DU PETROLE, 92502 Rueil-Malmaison (FR)
(72) Inventeur: Pucci, Annick, F-78290 Croissy sur Seine (FR); Mikitenko, Paul, F-78590 Noisy le Roi (FR); Zuliani, Massimo, F-92500 Rueil Malmaison (FR)

(56) Documents cités:
- US-A- 2 721 222
- US-A- 4 440 963

## Description

L'invention concerne la fabrication de l'éthyl tertiobutyl éther (en abrégé ETBE).

On sait que l'éthyl tertiobutyl éther, tout comme le méthyl tertiobutyl éther (en abrégé MTBE), peut être utilisé comme additif à haut indice d'octane pour les essences sans plomb ou à teneur en plomb réduite. On peut prévoir d'ajouter l'ETBE aux essences à des concentrations allant par exemple jusqu'à environ 15 % en volume.

Un procédé de fabrication du MTBE consiste à réaliser une réaction d'addition de méthanol sur de l'isobutène, par exemple contenu dans une coupe C₄ de vapocraquage, de craquage catalytique ou de déshydrogénation d'isobutane. Après réaction, le méthanol résiduel est en général séparé par distillation azéotropique avec les hydrocarbures en C₄, ce qui permet d'obtenir assez aisément le MTBE avec un degré de pureté convenable pour être additionné aux essences.

La fabrication de l'ETBE peut être réalisée par un procédé analogue, dans lequel l'éthanol remplace le méthanol. Un tel procédé est décrit par exemple dans "l'ETBE, un avenir pour l'éthanol" de A. FORESTIERE, B. TORCK et G. PLUCHE, communication faite à la Conférence sur la Biomasse pour l'Energie et l'Industrie, Lisbonne, 9-13 Octobre 1989 et dans "MTBE/ETBE, an Incentive Flexibility for Refiners" par A. FORESTIERE et coll., communication faite à la "Conference on Oxygenated Fuels in Europe", Londres, 22-23 Mai 1990.

Cependant, par un tel procédé, contrairement au cas du MTBE, après élimination des hydrocarbures en C₄, la quasi-totalité de l'éthanol résiduel se retrouve en mélange avec l'ETBE produit. L'existence d'un azéotrope éthanol-ETBE à 21 % en poids d'éthanol à la pression atmosphérique, bouillant à 66,6 °C, rend difficile la séparation de l'ETBE avec un degré de pureté suffisant pour satisfaire les spécifications concernant la teneur en éthanol des essences. Ainsi la teneur en éthanol de l'ETBE doit être généralement comprise entre 0,5 et 10 % en poids. Avantageusement, l'ETBE devra être purifié à moins de 2 % en poids d'éthanol pour être acheminé à la raffinerie.

Ainsi, pour que l'ETBE puisse concurrencer le MTBE comme additif améliorant l'indice d'octane des essences sans plomb, il était particulièrement souhaité de trouver un procédé de séparation économiquement attractif. C'est ce que l'invention propose.

L'invention a donc pour objet un procédé de séparation de l'ETBE à partir de mélanges qu'il forme avec l'éthanol, et plus particulièrement à partir des mélanges ETBE-éthanol issus de la réaction de l'éthanol sur une coupe C₄ de vapocraquage, de craquage catalytique ou de déshydrogénation d'isobutane.

L'invention a également pour objet un procédé de fabrication de l'ETBE incluant une telle opération de séparation et dans lequel l'éthanol est recyclé au réacteur d'éthérification.

Le procédé de séparation de l'ETBE de l'invention s'adresse en général aux mélanges constitués essentiellement d'éthanol et d'ETBE en proportions variées, et plus particulièrement aux mélanges issus de la réaction d'addition d'éthanol sur l'isobutène contenu dans une coupe C₄ de vapocraquage, de craquage catalytique ou de déshydrogénation d'isobutane, qui contiennent en général d'environ 5 à 50 %, le plus souvent de 10 à 30 %, en poids d'éthanol. Ils peuvent contenir en outre de très faibles proportions d'autres constituants, essentiellement des dimères de l'isobutène, tels que les triméthylpentènes, de l'alcool butylique tertiaire, du diéthyléther et des hydrocarbures en C₅ ; ils peuvent contenir également des traces d'éthers provenant de l'addition d'éthanol sur les oléfines en C4 autres que l'isobutène ; dans la présente description ces éthers ne seront pas distingués de l'ETBE

Le procédé de séparation de l'ETBE de l'invention, basé sur une distillation hétéroazéotropique mettant en jeu de l'eau comme tiers corps entraîneur, met en oeuvre deux colonnes couplées avec un décanteur de tête. L'éthanol purifié est recueilli en fond de première colonne et l'ETBE purifié, en fond de deuxième colonne.

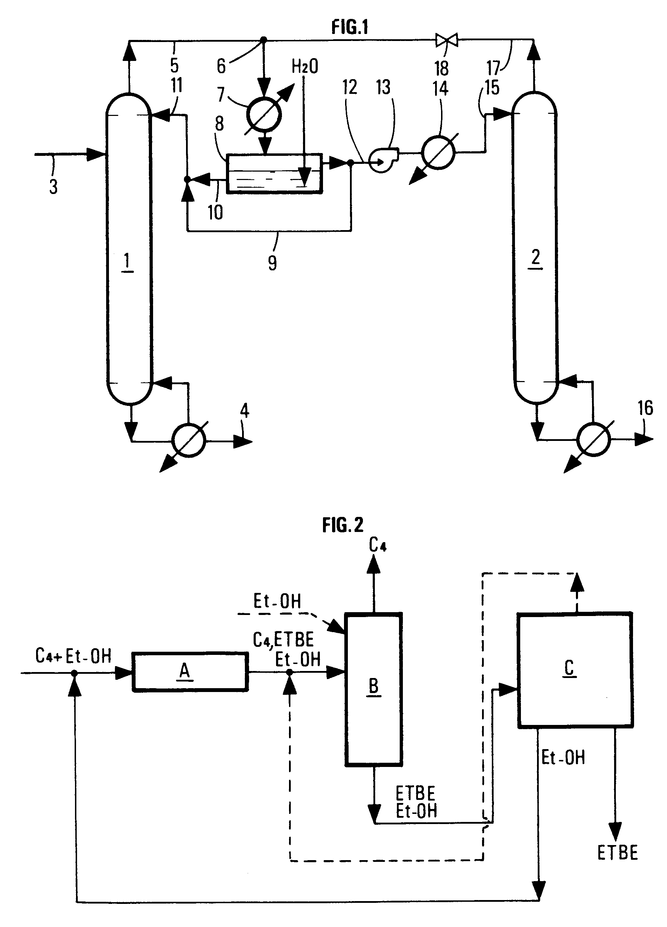

Le procédé de l'invention est décrit ci-après en relation avec la Figure 1, dont l'agencement, représenté de manière schématique, montre notamment les deux colonnes (1 et 2) et le décanteur (8), ainsi que les lignes qui les relient.

L'eau mise en jeu dans le procédé de l'invention peut être au moins en partie introduite dans l'installation avant son démarrage, par exemple dans le décanteur, la quantité complémentaire éventuellement nécessaire pouvant être ensuite introduite progressivement, pendant une phase transitoire, jusqu'à ce que soient établies les conditions d'équilibre caractérisant le régime stationnaire du procédé, telles qu'elles apparaîtront dans la description qui suit. Cette introduction progressive d'eau peut être effectuée par exemple au niveau du décanteur.

La charge consistant en le mélange contenant de l'ETBE et de l'éthanol à séparer est introduite par la ligne 3 dans la partie supérieure d'une première colonne 1, opérant sous une pression p₁, en général voisine de la pression atmosphérique et qui peut être par exemple de 0,05 à 0,2 MPa, et chauffée à une température de fond de 60 à 95 °C. La température d'introduction de la charge peut être selon le cas de 50 à 80 °C.

Le résidu sortant en fond par la ligne 4 consiste en éthanol purifié.

Le distillat sort en tête par la ligne 5 à une température voisine de celle de l'azéotrope ternaire eau/éthanol/ETBE à la pression p₁. Sa composition est voisine de celle de cet azéotrope à la pression p₁ ; par exemple pour une pression p₁ de 0,1 MPa, elle est d'environ 82,9 % en poids d'ETBE, environ 11,6 % en poids d'éthanol et environ 5,50 % en poids d'eau. Ce distillat est mélangé en 6 au distillat de la deuxième colonne, appauvri en ETBE, comme cela sera décrit plus loin. Le mélange obtenu est condensé dans le condenseur 7 et recueilli dans le décanteur 8, dans lequel deux phases se séparent :
- la phase inférieure contient une proportion prépondérante d'eau, une proportion mineure d'éthanol et quelques pourcents d'ETBE ;
- la phase supérieure contient une proportion prépondérante d'ETBE, une proportion mineure d'éthanol et quelques pourcents d'eau.

Pour assurer en tête de la colonne 1 un reflux liquide ayant une composition voisine de celle de l'azéotrope ternaire à la pression p₁, on fait sortir du décanteur 8 par la ligne 9 un flux de phase supérieure (riche en ETBE), auquel on adjoint un flux de phase inférieure aqueuse sortant du décanteur par la ligne 10, les débits desdits flux, compte tenu des compositions respectives des deux phases, étant réglés de telle manière que le rapport pondéral du débit de phase aqueuse au débit de phase ETBE soit de 0,035 à 0,040. Ces flux sont envoyés en tête de la colonne 1, en 11, à une température de 60 à 70 °C.

De façon alternative, le reflux nécessaire d'une partie de la phase aqueuse pourrait également être généré en tête de la colonne 1 par exemple par un condenseur partiel interne à la colonne, ou encore provoqué par un sousrefroidissement du reflux de phase ETBE au-dessous de son point de bulle.

Un second flux de phase supérieure riche en ETBE sort du décanteur 8 par la ligne 12. Il est porté par la pompe 13 à une pression p₂, supérieure à la pression p₁ en général d'une valeur Δp d'environ 0,4 à 1 MPa, et chauffé dans l'échangeur 14 à une température de 100 à 120 °C, avant d'être envoyé par la ligne 15 à la partie supérieure de la colonne 2 qui opère à la pression p₂ avec une température de fond de 120 à 150 °C.

La mise en oeuvre de la colonne 2 à une pression supérieure à celle de la colonne 1 permet notamment de réduire le taux de rebouillage de ladite colonne 2 d'un facteur pouvant aller de 5 à 10, ce qui représente un avantage considérable du point de vue économique.

Par ailleurs, on a observé que la mise en oeuvre de la colonne 2 sous une pression trop élevée présentait, outre l'inconvénient de rendre plus difficile la séparation des produits, celui du risque de dégradation thermique de l'ETBE au rebouillage. Aussi, la pression p₂ ne dépasse pas en général 1 MPa. Elle est le plus souvent d'environ 0,5 MPa.

Pour réaliser l'écart de pression Δp désiré, on peut envisager de fixer une pression p₁ inférieure à la pression atmosphérique. Cependant, cette solution nécessite l'installation supplémentaire d'un système de maintien de vide, ce qui n'est pas avantageux du point de vue économique. Aussi, la pression p₁ est de préférence de 0,1 à 0,2 MPa.

Le résidu de colonne 2 sortant par la ligne 16 à une température de 100 à 120 °C consiste essentiellement en ETBE purifié.

Le distillat sortant en tête par la ligne 17, appauvri en ETBE, peut être constitué par exemple de 72 à 76 % en poids d'ETBE, 18 à 20 % en poids d'éthanol et 5 à 7 % en poids d'eau.

Il est détendu à travers la vanne de détente 18 jusqu'à la pression p₁ du distillat de la colonne 1, auquel il est mélangé en 6. Le mélange des distillats est ensuite condensé en 7 pour être recueilli dans le décanteur 8, comme déjà décrit plus haut.

Dans les conditions opératoires mentionnées dans la description qui précède, l'éthanol en fond de colonne 1 et l'ETBE en fond de colonne 2 peuvent être obtenus avec des degrés de pureté élevés, par exemple de plus de 98 % en poids pour l'éthanol et de 99,99 % en poids pour l'ETBE.

Les pertes d'eau qui peuvent survenir pendant la mise en oeuvre du procédé (sous forme de traces d'eau sortant éventuellement avec l'éthanol et/ou avec l'éthyl tertiobutyl éther) peuvent être compensées par un appoint, par exemple périodique, effectué par exemple au niveau du décanteur.

L'invention propose également un procédé de fabrication d'ETBE par éthérification au moyen d'éthanol de l'isobutène contenu dans une coupe C₄ de vapocraquage, de craquage catalytique ou de déshydrogénation d'isobutane.

Le procédé de fabrication d'ETBE, décrit en liaison avec le schéma de la Figure 2 annexée, comprend alors les étapes suivantes : dans une zone A, on met en contact dans des conditions de réaction de l'éthanol et une coupe C₄ de vapocraquage, de craquage catalytique ou de déshydrogénation d'isobutane ; le produit issu de la zone réactionnelle A contient principalement de l'ETBE, de l'éthanol, des hydrocarbures en C₄ autres que l'isobutène et de l'isobutène non réagi. Ce produit est envoyé dans une zone de distillation B, où sont séparés en tête les hydrocarbures en C₄ très appauvris en isobutène et, en fond, un mélange d'ETBE et d'éthanol. Si l'on souhaite une élimination plus complète de l'isobutène, on peut mettre en oeuvre, au lieu d'une simple distillation B, une distillation réactive B', mettant en jeu un appoint d'éthanol, comme indiqué en pointillés sur la Figure 2. Dans tous les cas, le mélange d'ETBE et d'éthanol, recueilli en fond de zone B est envoyé dans une zone C, où est réalisé le procédé de séparation de l'invention.

A partir de la zone C, l'éthanol purifié recueilli est avantageusement recyclé comme appoint vers la zone de réaction A et/ou vers la zone de distillation réactive B'.

L'exemple suivant illustre l'invention.

### EXEMPLE

La charge à traiter contient 80 % en poids d'ETBE et 20 % en poids d'éthanol.

On utilise :
- une première colonne à distiller en acier inoxydable de 50 mm de diamètre et comportant 38 plateaux perforés à déversoir espacés de 5 cm ;
- une seconde colonne à distiller en acier inoxydable de 50 mm de diamètre et comportant 15 plateaux perforés à déversoir espacés de 5 cm ; et
- un décanteur de 0,5 litre.

Ces appareils sont agencés comme indiqué sur le schéma de la figure annexée.

On introduit au départ 0,100 kg d'eau dans le décanteur.

La charge est introduite dans la première colonne au niveau du 2e plateau (les plateaux étant comptés de haut en bas).

La première colonne est chauffée à une température de fond de 78 °C et la seconde colonne à une température de fond de 135 °C.

Durant la période de mise en régime, on effectue une introduction progressive d'eau au niveau du décanteur jusqu'à ce que soient établis les équilibres caractérisant le régime stationnaire dont les conditions sont regroupées dans le tableau suivant.

**TABLEAU**

| | Colonne 1 | Colonne 2 |
|---|---|---|
| Pression (MPa) | 0,1 | 0,5 |

| Alimentation | | |
|---|---|---|
| Débit (kg/h) | 1,50 | 4,47 |
| Température (°C) | 67 | 113 |

| Distillat | | |
|---|---|---|
| Débit (kg/h) | 5,78 | 3,28 |
| Température (°C) | 64 | 114 |

| Produit | | |
|---|---|---|
| Débit (kg/h) | 0,30 | 1,20 |
| Température (°C) | 77,5 | 64 |
| Pureté (% poids) | Ethanol 98,6 | ETBE >99,9 |

Les deux distillats sont réunis et leur mélange est condensé puis envoyé au décanteur (débit liquide à l'entrée : 9,06 kg/h).

Du décanteur, on envoie un reflux vers la première colonne au plateau n° 1 à 65 °C (débit total :4,58 kg/h) ce reflux résultant de la réunion d'un flux de phase supérieure (débit : 4,42 kg/h) et d'un flux de phase inférieure (débit : 0,16 kg/h) issus du décanteur.

Du décanteur, on envoie également un flux de phase supérieure qui est porté à 0,5 MPa et à 113 °C pour alimenter la colonne 2 au niveau du plateau n° 1.

Les débits, températures et puretés des produits son indiqués au tableau ci-dessus.

## Revendications

1. Procédé de séparation de l'éthyl tertiobutyl éther à partir de mélanges qu'il forme avec l'éthanol, caractérisé en ce qu'une charge comprenant principalement de l'éthyl tertiobutyl éther et de l'éthanol est traitée en continu dans une installation comprenant deux colonnes de distillation reliées en tête à un décanteur ; en ce que de l'eau est introduite dans ladite installation jusqu'à ce que la quantité introduite corresponde au fonctionnement en régime stationnaire dudit procédé ; et en ce que ladite charge est introduite à une température de 50 à 80 °C dans la partie supérieure de la première colonne qui fonctionne à une pression p₁ de 0,05 à 0,2 MPa, ladite première colonne étant chauffée à une température de fond de 60 à 95 °C, le distillat de ladite première colonne est mélangé au distillat de la seconde colonne, le mélange ainsi formé est condensé, le condensat obtenu est décanté en une phase inférieure comprenant une proportion prépondérante d'eau et une proportion mineure d'éthanol et en une phase supérieure comprenant une proportion prépondérante d'éthyl tertiobutyl éther et une proportion mineure d'éthanol, on assure en tête de ladite première colonne un reflux liquide ayant une composition voisine de celle de l'azéotrope ternaire à la pression p₁, en envoyant en tête de ladite première colonne, à une température de 60 à 70 °C, des proportions appropriées d'un premier flux de phase supérieure et d'un flux de phase inférieure, un second flux de phase supérieure, porté à une pression p₂ supérieure à la pression p₁ d'une valeur Δp de 0,4 à 1 MPa et chauffé à une température de 100 à 120 °C, alimente en sa partie supérieure ladite seconde colonne opérant sous la pression p₂ et chauffée à une température de fond de 120 à 150 °C, le distillat de ladite seconde colonne est détendu à la pression p₁, puis mélangé au distillat de ladite première colonne, l'éthanol purifié est recueilli en fond de première colonne et l'éthyl tertiobutyl éther purifié, en fond de seconde colonne.

2. Procédé selon la revendication 1, caractérisé en ce que la charge comprend de 5 à 50 % en poids d'éthanol.

3. Procédé selon la revendication 1, caractérisé en ce que la charge comprend de 10 à 30 % en poids d'éthanol.

4. Procédé selon l'une des revendications 1 à 3, caractérisé en ce que la pression p₁ est de 0,1 à 0,2 MPa et la pression p₂ est au plus de 1 MPa.

5. Procédé selon l'une des revendications 1 à 4, caractérisé en ce que ledit flux de phase inférieure et ledit premièr flux de phase supérieure du décanteur sont dans un rapport pondéral allant de 0,035 à 0,040.

6. Procédé selon l'une des revendications 1 à 5, caractérisé en ce que la charge provient d'une fabrication d'ETBE par éthérification par de l'éthanol de l'isobutène contenu dans une coupe C₄ de vapocraquage, de craquage catalytique ou de déshydrogénation d'isobutane.

7. Procédé selon la revendication 6, caractérisé en ce que la charge renferme environ 80 % en poids d'ETBE et environ 20 % en poids d'éthanol.

8. Procédé de fabrication d'éthyl tertiobutyl éther par éthérification par de l'éthanol de l'isobutène contenu dans une coupe C₄ de vapocraquage, de craquage catalytique ou de déshydrogénation d'isobutane, dans lequel, dans une zone de réaction A, on met en contact dans des conditions réactionnelles de l'éthanol et ladite coupe C₄ ; le produit issu de ladite zone de réaction A, contenant principalement de l'ETBE, de l'éthanol, des hydrocarbures en C₄ autres que l'isobutène et de l'isobutène non réagi, est envoyé dans une zone de distillation B où sont séparés en tête les hydrocarbures en C₄ incluant l'isobutène non réagi et en fond un mélange d'ETBE et d'éthanol ; on envoie ledit mélange dans une zone de séparation C ; ledit procédé étant caractérisé en ce que, dans ladite zone de séparation C, on met en oeuvre un procédé selon l'une des revendications 1 à 7.

9. Procédé selon la revendication 8, caractérisé en ce que ladite zone de distillation B est remplacée par une zone de distillation réactive B', dans laquelle on introduit de l'éthanol pour éliminer l'isobutène non réagi.

10. Procédé selon l'une des revendications 8 et 9, caractérisé en ce que l'éthanol purifié issu de la zone de séparation C est recyclé à l'entrée de la zone de réaction A et/ou vers la zone de distillation B'.

## Claims

1. A method of separating ethyl tertiobutyl ether from mixtures which it forms with ethanol, characterised in that a charge chiefly comprising ethyl tertiobutyl ether and ethanol is treated continuously in an installation comprising two distillation columns connected overhead to a decanter; that water is introduced into said installation until the quantity introduced corresponds to operation of said method in a stationary state; and that the charge is introduced at a temperature of 50 to 80°C into the upper part of the first column, which operates at a pressure p1 of 0.05 to 0.2 MPa, said first column being heated to a bottom temperature of 60 to 95°C; the distillate from the first column is mixed with that from the second column and the mixture thus formed is condensed; the condensate obtained is decanted into a lower phase with a major proportion of water and a minor proportion of ethanol, and an upper phase with a major proportion of ethyl tertiobutyl ether and a minor proportion of ethanol; a liquid reflux of a composition close to that of the ternary azeotrope at pressure p1 is produced at the top of the first column, by passing, appropriate proportions of a first stream of upper phase and of a stream of lower phase to the top of the first column, at a temperature of about 60 to 70°C; a second stream of upper phase, brought to a pressure p2, which is higher than pressure p1 by a value Δp of 0.4 to 1 MPa, and heated to a temperature of 100 to 120°C, is fed to the upper part of the second column, which operates at pressure p2 and which is heated to a bottom temperature of 120 to 150°C; the distillate from the second column is depressurised to pressure p1 then mixed with the distillate from the first column; and the purified ethanol is collected from the bottom of the first column and the purified ethyl tertiobutyl ether from the bottom of the second column.

2. The method of claim 1, characterised in that the charge includes 5 to 50% by weight of ethanol.

3. The method of claim 1, characterised in that the charge includes 10 to 30% by weight of ethanol.

4. The method of any of claims 1 to 3, characterised in that the pressure p1 is from 0.1 to 0.2 MPa, and the pressure p2 is 1 MPa at the maximum.

5. The method of any of claims 1 to 4, characterised in that the stream of lower phase and the first stream of upper phase from the decanter are in a weight ratio ranging from 0.035 to 0.040.

6. The method of any of claims 1 to 5, characterised in that the charge emanates from preparation of ETBE through etherification of isobutene contained in a C₄ cut from steam cracking, catalytic cracking or dehydrogenation of isobutane, by ethanol.

7. The method of claim 6, characterised in that the charge contains about 80% by weight of ETBE and about 20% by weight of ethanol.

8. A method of preparing ethyl tertiobutyl ether through etherification of isobutene contained in a C₄ cut from steam cracking, catalytic cracking or dehydrogenation of isobutane, by ethanol wherein, in a reaction zone A, ethanol and the C₄ cut are put into contact under reaction conditions; the product discharged from the reaction zone A, chiefly containing ETBE, ethanol, C₄ hydrocarbons other than isobutene and non-reacted isobutene, is passed into a distillation zone B, where the C₄ hydrocarbons including non-reacted isobutene are separated at the top and a mixture of ETBE and ethanol at the bottom; and said mixture is passed into a separating zone C; characterised in that the method of any of claims 1 to 7 is carried out in said separating zone C.

9. The method of claim 8, characterised in that the distillation zone B is replaced by a reactive distillation zone B', into which ethanol is introduced to remove the non-reacted isobutene.

10. The method of claim 8 or 9, characterised in that the purified ethanol from the separating zone C is recycled to the inlet of the reaction zone A and/or to the distillation zone B'.

## Patentansprüche

1. Verfahren zur Trennung von Ethyltertbutylether auf der Grundlage von Mischungen, die er mit Ethanol bildet, dadurch gekennzeichnet, daß eine Charge, die im wesentlichen den Ethyltertbutylether und das Ethanol umfaßt, kontinuierlich in einer Einrichtung behandelt wird, welche Zwei am Kopf mit einem Dekantiergefäß verbundene Kolonnen zur Destillation umfaßt, daß Wasser in diese Einrichtung eingeführt wird, bis die eingeführte Menge dem Betrieb des Verfahrens im stationären Zustand entspricht, und daß die Menge mit einer Temperatur von 50 bis 80°C in den oberen Bereich der ersten Kolonne, die bei einem Druck p₁ von 0,05 bis 0,2 mPa arbeitet, eingeführt wird, wobei die erste Kolonne auf eine Bodentemperatur von 60 bis 95°C erwärmt wird, das Destillat der ersten Kolonne mit dem Dastillat der zweiten Kolonne gemischt wird, die so gebildete Mischung kondensiert wird, das erhaltene Kondensat in eine untere Phase, die ein vorherrschendes Verhältnis an Wasser und ein kleineres Verhältnie an Ethanol umfaßt, und in eine obere Phase, die ein vorherrschendes Verhältnis an Ethyltertbutylether und ein kleineres Verhältnis an Ethanol umfaßt, dekantiert wird, am Kopf der ersten Kolonne ein flüssiger Rückfluß, der eine zu derjenigen des ternären Azeotrops beim Druck p₁ benachbarte Zusammensetzung aufweist, durch Beschicken der ersten Kolonne am Kopf mit geeigneten Verhältnissen eines ersten Flusses der oberen Phase und eines Flusses der unteren Phase bei einer Temperatur von 60 bis 70°C gesichert wird, ein zweiter Fluß der oberen Phase , der auf einen zu dem Druck p₁ größeren Druck p₂ eines Wertes Δp Von 0,4 bis 1 mPa gebracht und auf eine Temperatur von 100 bis 120°C erwärmt wird, dem oberen Bereich der zweiten Kolonne, die unter dem Druck p₂ arbeitet und auf eine Bodentemperatur von 120 bis 150°C erwärmt ist, zugefürt wird, das Destillat der zweiten Kolonne auf den Druck p₁ entspannt, dann mit dem Destillat der ersten Kolonne gemischt wird, das reine Ethanol am Boden der ersten Kolonne und der reine Ethyltertbutylether am Boden der zweiten Kolonne gesammelt wird.

2. Verfahren nach Anspruch 1, dadurch gekennzeichnet, daß die Charge 5 bis 50 Gew.-% Ethanol umfaßt.

3. Verfahren nach Anspruch 1, dadurch gekennzeichnet, daß die Charge 10 bis 30 Gew.-% Ethanol umfaßt.

4. Verfahren nach einem der Ansprüche 1 bis 3, dadurch gekennzeichnet, daß dar Druck p₁ 0,1 bis 0,2 mPa beträgt und der Druck p₂ höchstens 1 mPa beträgt.

5. Verfahren nach einem der Ansprüche 1 bis 4, dadurch gekennzeichnet, daß der Fluß der unteren Phase und der erste Fluß der oberen Phase des Dekantiergafäßes in einem Gewichtsverhältnis, das sich von 0,035 bis 0,040 bewegt, stehen.

6. Verfahren nach einem der Ansprüche 1 bis 5, dadurch gekennzeichnet, daß die Charge aus einer Herstellung von ETBE durch Veretherung von Isobuten mittels Ethanol stammt, das in einem C₄-Schnitt eines Dampfcrackens, eines katalytischen Crackens oder einer Dehydrierung von Isobutan enthalten ist.

7. Verfahren nach Anspruch 6, dadurch gekennzeichnet, daß die Charge etwa 80 Gew.-% ETBE und ungefähr 20 Gew.-% Ethanol einschließt.

8. Verfahren der Herstellung von Ethyltertbutylether durch Veretherung von Isobuten mittels Ethanol, das in einem C₄-Schnitt eines Dampfcrackens, eines katalytischen Crackens oder einer Dehydrierung von Isobutan enthalten ist, bei welchem man des Ethanol und den C₄-Schnitt in einer Reaktionszone A unter Reaktionsbedingungen in Kontakt bringt, das aus der Reaktionszone A stammende Produkt, das im wesentlichen den ETBE, das Ethanol, von Isobuten unterschiedliche C₄-Kohlenwasserstoffe und das keine Reaktion eingegangenne Isobuten enthält, in eine Destillationszone B eingebracht wird, in welcher am Kopf die C₄-Kohlenwasserstoffe, die das keine Reaktion eingegangene Isobuten einschließen, und am Boden eine Mischung aus ETBE und aus Ethanol getrennt werden, man die Mischung in eine Trennzone C verbringt, wobei das Verfahren dadurch gekennzeichnet ist, daß man in der Trennzone C ein Verfahren gemäß einem der Ansprüche 1 bis 7 durchführt.

9. Verfahren nach Anspruch 8, dadurch gekennzeichnet, daß die Destillationszone B durch eine reaktive Destillationszone B' ersetzt wird, in welcher man das Ethanol einführt, um das keine Reaktion eingegangene Isobuten zu entfernen.

10. Verfahren nach einem der Ansprüche 8 und 9, dadurch gekennzeichnet, daß reines, aus der Trennzone C stammendes Ethanol zum Einlaß der Reaktionszone A und/oder zur Destillationszone B' zurückgeführt wird.
